(19) Europäisches Patentamt
European Patent Office
Office européen des brevets

(11) Publication number: 0 279 241 A1

# (12) EUROPEAN PATENT APPLICATION

(21) Application number: 88101205.8

(51) Int. Cl.[4]: A61K 31/405 , A61K 47/00

(22) Date of filing: 27.01.88

(30) Priority: 28.01.87 US 7683

(43) Date of publication of application:
24.08.88 Bulletin 88/34

(84) Designated Contracting States:
AT BE CH DE ES FR GB GR IT LI LU NL SE

(71) Applicant: WARNER-LAMBERT COMPANY
201 Tabor Road
Morris Plains New Jersey 07950(US)

(72) Inventor: Balwani, Gul P.
25-294 Alpine Way
Woodbridge New Jersey 07095(US)
Inventor: Hu, Jeannie C.
197 N. Livingston Avenue
Livingston New Jersey 07039(US)
Inventor: Sampson, Karla J.
15 Elmwood Terrace
West Caldwell New Jersey 07006(US)

(74) Representative: Mansmann, Ivo
c/o Gödecke AG - Patentabteilung Postfach
569 - Mooswaldallee 1-9
D-7800 Freiburg(DE)

## (54) Stabilized ophthalmic compositions.

(57) This invention describes ophthalmic compositions containing an antibiotic in a stable solution and a process for preparing these compositions.

EP 0 279 241 A1

Xerox Copy Centre

## STABILIZED OPTHALMIC COMPOSITIONS

The present invention relates to ophthalmic compositions containing an antibiotic in a stable solution. The antibiotic is enoxacin, a naphthyridine compound having high antibacterial activity against gram-positive bacteria and gram-negative bacteria.

United States patent 4,359,578 covers the compound enoxacin. The sesquihydrate of the compound is coverd in United States patent 4,442,101. A composition and an oral administration of the compound are covered in United States patent 4,352,803. United States patent 4,551,456 covers a method of treating ocular bacterial infections using norfloxacin, ofloxacin, perfloxacin, AT2266 (enoxacin and Bayer 09867. The latter patent is incorporated herein by reference. The present invention relates to stabilized, non-irritating compositions useful in the treatment of bacterial infections. These compositions have pased the USP preservative effectiveness test. These compositions have been shown to be non-irritating in a procedure outlined in the US-Code of Federal Regulations for testing eye irritations (16 CFR 1500.42).

The present invention relates to stabilized, non-irritating compositions for the treatment and prevention of bacterial infections of the eye. The compositions contain antibacterially effective amounts of enoxacin, an acetate buffer, a preservative, glycerin, a viscosity building agent and acid or base to adjust the pH.

The invention further relates to a method of treating ocular bacterial infections.

The invention also relates to a method for preparing a stable, non-irritating composition for treating ocular bacterial infections.

The novel compositions of this invention comprise an active ingredient, enoxacin, 1-ethyl-6-fluoro-1,4-dihydro-4-oxo-7-(1-piperazinyl)-1,8-naphthyridine-3-carboxylic acid. USA-4,359,578 covers a preparation of the active ingredient. The novel compositions include the hydrates, solvates or ophthalmologically acceptable salts thereof including acid or base addition salts.

The acids may be various organic and inorganic acids such as hydrochloric acid, acetic acid, lactic acid, succinic acid, galacturonic, aspartic, gluconic, glutamic, lactobionic acid and methansulfonic acid and the like.

The bases may be any inorganic or organic bases capable of forming salts with the carboxylic group of the compound such as sodium or potassium hydroxide or sodium or potassium carbonate and the like.

All percentages herein recited are weight percentages based on the total composition unless otherwise stated.

Compositions of the present invention may contain from about 0.05% to about 3.0% of enoxacin. Preferred compositions of the present invention contain from about 0.10% to about 1.0% of the compound. Unit dosage is from between 0.01 mg to 2.0 mg, and preferably .05 mg to 1.0 mg. Especially preferred is 0.1 mg to 0.5 mg of the compound.

The administration of the unit dosage is as frequently as necessary. As with all eye medication dosages vary and must be individualized on the basis of the disease and responses of the patient.

The composition can, if desired, also contain other therapeutic agents. The percentage of the active ingredient in the foregoing compositions can be varied within wide limits but for practical purposes is preferably present in a concentration of at least 2% in a primarily liquid composition. The most satisfactory compositions are those in which a much higher proportion of active ingredient is present.

In a typical method of preparation the polyvinyl alcohol resin is added to water and is mixed until the alcohol is dissolved. The acetic acid, anhydrous sodium acetate, benzalkonium chloride, and glycerin are added and mixed until all dissolve. The compound, enoxacin (AT 2266), is then added and mixed until it dissolves. The pH is checked and adjusted if necessary with acid or base to a pH of about 4 to 6, preferably to about 5. Most preferred is 5.0 ± 0.2. Sufficient water for injection is added to bring the volume to required quantity and the pH is again checked and adjusted if necessary.

In a preferred method of preparation of the stabilized, non-irritating composition, polyvinyl alcohol resin 20-90 is added to water and mixed until the alcohol dissolves. Then acetic acid, anhydrous sodium acetate, benzalkonium chloride, and glycerin are all added and mixed until all are dissolved. To that enoxacin is added and mixed until it dissolves. Then the pH of the mixture is adjusted with hydrochloric acid and/or sodium hydroxide to from about 4.7 to about 5.3. After that adjustment the solution is brought up to volume and the pH checked and adjusted, if necessary, to pH 5 ± 0.2.

The resulting solution is then sterilized by filtration through a sterilized filtration assembly fitted with, for example, a Millipore AW19 prefilter and a Millipore GVWP 0.22 µm sterilizing filter with an AP32 mesh spacer between the filters, using high purity nitrogen gas for positive pressure. Then 15 mls of the solution are aseptically filled into previously washed and sterilized bottles; plugs are aseptically inserted and the bottles are capped.

Formulations of these compounds may contain from about 0.05 to 3% and especially 0.10 to 1.0% of medicament although higher or lower dosages can be employed. As a unit dosage form between 0.01 to 2.0 mg, preferably 0.05 to 1.0 mg, and especially 0.1 to 0.5 mg of the compound is generally applied to the human eye, and can be administered as frequently as necessary.

These hereinbefore described dosage values are believed accurate for human patients and are based on the known and presently understood pharmacology of the compounds, and the action of other similar entities in the human eye. They reflect the best mode known. As with all medications, dosage requirements are variable and must be individualized on the basis of the disease and the response of the patient.

In the pharmaceutical preparation the active compound conveniently is admixed with a nontoxic pharmaceutical inorganic carrier. Typical of pharmaceutically acceptable carriers are, for example, water, mixtures of water and water-miscible solvents, such as lower alkanols or aralkanols, vegetable oils, polyalkylene glycols, petroleum based jelly, ethyl cellulose, ethyl oleate, carboxymethyl-cellulose, polyvinylpyrrolidone, isopropyl myristate and other conventionally employed acceptable carriers. The ophthalmological preparation may also contain nontoxic auxiliary substances such as emulsifying, preserving, wetting agents, bodying agents and the like, as for example, polyethylene glycols 200, 300, 400, and 600, carbowaxes 1,000, 1,500, 4,000 6,000, and 10,000, other antibacterial components such as quaternary ammonium compounds, EDTA and its salts phenylmercuric salts known to have cold sterilizing properties and which are noninjurious in use, thimerosal, methyl and propyl paraben, benzyl alcohol, phenylethanol, buffering ingredients such as sodium chloride, sodium borate, sodium acetate, gluconate buffers, and other conventional ingredients such as sorbitan monolaurate, triethanolamine, oleate, polyoxyethylene sorbitan monopalmitylate, dioctyl sodium sulfosuccinate, monothioglycerol, thiosorbitol, ehtylenediamine tetraacetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like. The pharmaceutical preparation may also be in the form of a solid insert.

While many patients find liquid medication to be entirely satisfactory, others may prefer a solid medicament that is topically applied to the eye, for example, a solid dosage form that is suitable for insertion into the cul-de-sac. To this end of the antibiotic can be included with a nonbioerodible insert, i.e., one which after dispensing the drug remains essentially intact, or a bioerodible insert, i.e., one that either is soluble in lacrimal fluids, or otherwise disintegrates. While the insert employed is not critical and those disclosed in U.S. patent numbers 3,650,200 Higuchi; 3,811,444 Heller et al; 4,177,256 Michaels, et al; 3,868,445 Ryde, et al; 3,845,201 Haddad; 3,981,303 Higuchi; and 3,867,519 Michaels, are satisfactory; in general, however, the inserts described below are found preferable.

For example, one may use a solid water soluble polymer as the carrier for the medicament. The polymer used to form the insert may be any water soluble nontoxic polymer, for example, cellulose derivatives such as methylcellulose, sodium carboxymethyl cellulose, or a hydroxy lower alkyl cellulose such as hydroxyethyl cellulose, hydroxypropyl cellulose, hydroxypropylmethyl cellulose and the lke; acrylates such as polyacrylic acid salts, ethyl acrylates, polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum, and mixtures of said polymer.

The ophthalmic formulation may also be in the form of a clear physiologically-acceptable liquid which forms a semi-solid "gel" at human body temperatures. Polymers having these properties are tetra substituted derivatives of ethylene diamine (poloxamine, w=2 in Formula I), propylene diamine (w=3), butylene diamine (w=4), pentylene diamine (w=5) or hexylene diamine (w=6). The substituents are block copolymers of poly(oxypropylene) and poly(oxyethylene) of various chain lengths and ratios X to y in the general formula of the polymer shown below

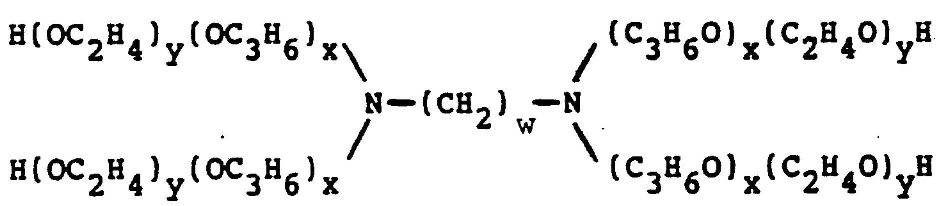

I

wherein w is an integer from two through six.

A typical polymer system would contain a polymer containing approximately 40 to 80% poly-(oxypropylene). The total molecular weight of the polymer used is at a minimum about 7,000 and can go as high as 50,000 but preferably in the range of 7,000 to 30,000 and x and y are any integers within the above constraints. Preferred polymers are those of the formula above where w = 2, namely the poloxamine polymer.

The aqueous drug delivery vehicle would contain from 10% to 50% by weight of the entire vehicle as polymer described above. The aqueous drug delivery vehicle would also contain the drug of therapeutic agent in addition to various additives such as acids or based to adjust the pH of the composition, buffers to maintain the pH, preservatives to control bacterial contamination, other additives to provide for drug solubility and stability and formulation performance with purified water making up the remainder of the drug delivery vehicle.

The ophthalmic formulation may also be in form of stable plurilamellar vesicles which comprise the antibiotic active ingredient encapsulated with liposomes as described in U.S. patent number 4,552,803.

Ophthalmic inserts are manufactured from compression molded films which are prepared on a Carver Press by subjecting the powdered mixture of the above ingredients to a compressional force of 12,000 pounds (gauge) at 300°F for one to four minutes. The film is cooled under pressure by having cold water circuits in the platen. Ophthalmic inserts are then individually cut from the film with a rod-shaped punch. Each insert is placed into a vial, which is then placed in a humidity cabinet (88% R.H. at 30°C) for two to four days. After removal from the humidity cabinet, the vial containing the hydrate insert are then autoclaved at 250°F for 0.5 hour.

The following example is illustrative of the present invention. It is not intended to restrict the scope of the invention.

Example I

Formulation of 0.3% Solution Ingredient
Enoxacin 0.3%
Acetate buffer 0.1 M
Benzalkonium chloride 0.008%
Glycerin 1.00%
Polyvinyl alcohol 20-90 1.4%
HCl/NaOH qs ad pH 5.0 ± 0.3

Reasonable variation of the above, such as those which would occur to a skilled artisan, can be made herein without departing from the scope of the instant invention.

## Claims

1. A stabilized ophthalmic composition in unit dosage form, which comprises :
one or more pharmaceutically acceptable excipients, and an amount of enoxacin or a hydrate, solvate, or an ophthalmotologically acceptable acid addition or base salt thereof effective to treat or prevent bacterial infections of the eye conditions in a warm-blooded animal to whom one or more unit doses of said composition are administered.

2. A composition according to Claim 1 wherein the amount of enoxacin is about 0.1 to about 0.6% by weight.

3. A composition according to Claim 1 and further containing an acetate buffer the amount of said buffer being from about 0.01 M to about 0.2 M by weight per cent.

4. A composition according to Claim 3 wherein the buffer comprises sodium acetate and acetic acid.

5. A composition according to Claim 1 and further containing a preservative which comprises benzalkonium chloride of about 0.001% to about 0.010% by weight.

6. A composition according to Claim 1 and further containing glycerin of about 0.10 to about 2.00% by weight.

7. A composition according to Claim 1 and further containing a viscosity building agent.

8. A composition according to Claim 7 wherein the viscosity building agent comprises polyvinyl alcohol resin 20-90 in the amount of about 0.4% to about 2.4% by weight.

9. A composition according to Claim 1 and further containing a pH buffer sufficient for maintaining the pH of the composition within the range of about 4.7 to about 5.3.

10. A pharmaceutical composition according to Claim 1 comprising about 0.25 to about 0.35% by weight of enoxacin about .09 M to about .11 M by weight acetate buffer, about 0.006 to about 0.010% by weight benzalkonium chloride, about 0.80% to about 1.2% by weight glycerin, about 1.1 to about 1.7% by weight polyvinyl alcohol resin, physiologically acceptable acid or base in an amount sufficient to bring the pH of the composition to between about 4.7 to about 5.3, and sufficient solution to maintain the pH of the composition within the range of about 4.7 to about 5.3.

11. A method of preparing stable ophthalmic compositions containing enoxacin which comprises:

(a) adding a polyvinyl alcohol resin to water and mixing until the alcohol dissolves;

(b) adding to and mixing with the mixture from step (a) an acetate buffer, a preservative, and glycerin until all are dissolved;

(c) adding and mixing until dissolved enoxacin to the mixture from step (b); and

(d) adjusting the mixture from step (c) to a pH of 5 ± 0.2 and bringing the mixture to volume and checking and adjusting if necessary the pH.

12. A method of preparing stable ophthalmic compositions contaiing enoxacin which comprises:

(a) adding a polyvinyl alcohol resin 20-90 to water and mixing until the alcohol dissolves;

(b) adding to and mixing to the above mixture acetic acid, anhydrous sodium acetate, benzalkonium chloride, and glycerin until all are dissolved;

(c) adding and mixing enoxacin to the mixture in step (b) until it is dissolved; and

(d) adjusting the mixture of step (c) to a pH of 5 ± 0.2, bring the mixture of step (c) to volume, checking and adjusting if necessary the pH.

Claims for the following contracting States: Es, Gr.

1.) A method of preparing a stabilized ophthalmic composition containing enoxacin and comprising one or more pharmaceutically acceptable excipients, and an amount of enoxacin or a hydrate, solvate, or an ophthalmotologically acceptable acid addition or base salt thereof effective to treat or prevent baceterial infections of the eye conditions in a warm-blooded animal to whom one or more unit doses of said composition are administered, which comprises the steps of:

(a) adding polyvinyl alcohol resin to water and mixing until the alcohol dissolves;

(b) adding to and mixing with the mixture from step (a) an acetate buffer, a preservative, and glycerin until all are dissolved;

(c) adding and mixing until dissolved enoxacin to the mixture from step (b): and

(d) adjusting the mixture from step (c) to a pH of 5 ±0.2 and bringing the mixture to volume and checking and adjusting if necessary the pH.

2.) A method of preparing a stabilized ophthalmic composition according to Claim 1 containing enoxacin and comprising one or more pharmaceutically acceptable excipients, and an amount of enoxacin or a hydrate, solvate, or an ophthalmotologically acceptable acid addition or base salt thereof effective to treat or prevent bacterial infections of the eye conditions in a warm-blooded animal to whom one or more unit doses of said composition are administered, which comprises the steps of:

(a) adding a polyvinyl alcohol resin 20-90 to water and mixing until the alcohol dissolves;

(b) adding to and mixing to the above mixture acetic acid, anhydrous sodium acetate, benzalkonium chloride, and glycerin until all are dissolved;

(c) adding and mixing enoxacin to the mixture in step (b) until it is dissolved; and

(d) adjusting the mixture of step (c) to a pH of 5 ± 0.2 bring the mixture of step (c) to volume, checking and adjusting if necessary the pH.

3.) A method of preparing a stabilized ophthalmic composition according to Claim 1 and 2, wherein the amount of enoxacin is about 0.1 to about 0.6 % by weight.

4.) A method of preparing a stabilized ophthalmic composition according to Claim 1 and 2 and further containing an acetate buffer the amount of said buffer being from about 0.01 M to about 0.2 M by weight per cent.

5.) A method of preparing a stabilized ophthalmic composition according to Claim 4 wherein the buffer comprises sodium acetate and acetic acid.

6.) A method of preparing a stabilized ophthalmic composition according to Claim 1 and 2 and further containing a preservative which comprises benzalkonium chloride of about 0.001 % to about 0.010 % by weight.

7.) A method of preparing a stabilized ophthalmic composition according to Claim 1 and 2 and further containing glycerin of about 0.10 to about 2.00 % by weight.

8.) A method of preparing a stabilized ophthalmic composition according to Claim 1 and 2 and further containing a viscosity building agent.

9.) A method of preparing a stabilized ophthalmic composition according to Claim 8 wherein the viscosity building agent comprises polyvinyl alcohol resin 20-90 in the amount of about 0.4 % to about 2.4 % by weight.

10) A method of preparing a stabilized ophthalmic composition according to Claim 1 and 2 and further containing a pH buffer sufficient for maintaining the pH of the composition within the range of about 4.7 to about 5.3.

11) A method of preparing a stabilized ophthalmic composition according to Claim 1 and 2 comprising about 0.25 to about 0.35 % by weight of enoxacin about .09 M to about .11 M by weight acetate buffer, about 0.006 to about 0.010 % by weight benzalkonium chloride, about 0.80 % to about 1.2 % weight glycerin, about 1.1 to about 1.7 % by weight polyvinyl alcohol resin, physiologically acceptable acid or base in an amount sufficient to bring the pH of the composition to between about 4.7 to about 5.3, and sufficient solution to maintain the pH of the composition within the range of about 4.7 to about 5.3.

European Patent Office

# EUROPEAN SEARCH REPORT

Application Number

EP 88 10 1205

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X<br><br>Y | EP-A-0 142 426 (MERCK & CO. INC.)<br>* Whole document * & US-A-4 551 456 (Cat. D)<br>--- | 1-5,9<br><br>6-8,10-12 | A 61 K 31/495<br>A 61 K 47/00 |
| Y | US-A-4 409 205 (C.D. SHIVELY)<br>* Column 2, lines 13-43; column 3, line 34 - column 4, line 3; column 4, line 57 - column 5, line 10 *<br>--- | 6-8,10-12 | |
| A | EP-A-0 009 425 (LABORATOIRE ROGER BELLON & DAINIPPON PHARMACEUTICAL CO.)<br>* Page 1, lines 1-12; page 2, line 31 - page 3, line 1; page 11, lines 24-28 * & US-A-4 352 803, & US-A-4 359 578 (Cat. D)<br>--- | 1 | |
| A | EP-A-0 056 420 (SCHERING CORP.)<br>* Page 2, lines 11-21; page 4, lines 1-8; page 5, line 21 - page 6, line 6; page 6, lines 12-20 *<br>--- | 7,8 | TECHNICAL FIELDS SEARCHED (Int. Cl.4) |
| A | CHEMICAL ABSTRACTS, vol. 76, no. 26, 26th June 1972, page 326, abstract no. 158294c, Columbus, Ohio, US; B. BEDO et al.: "Use of poly(vinyl alcohol) in pharmaceutical practice. I. Poly(vinyl alcohol) used in the preparation of ophthalmic solutions", & REV. MED. (TIRGUMURES) 1971, 17(3-4), 448-52<br>* Abstract *<br>----- | 7,8 | A 61 K<br>C 07 D |

The present search report has been drawn up for all claims

| Place of search | Date of completion of the search | Examiner |
|---|---|---|
| THE HAGUE | 16-06-1988 | MUELLNERS W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)